# EUROPEAN PATENT APPLICATION

(11) **EP 4 180 808 A1**
(43) Date of publication of application: **17.05.2023**
(21) Application number: 22205842.2
(22) Date of filing: 07.11.2022
(51) Int. Cl.: G01N 33/28

(54) **WEAR DETECTION SYSTEM WITH METAL DEBRIS SENSOR ASSEMBLY**

(30) Priority: 07.11.2021 US 202163276651 P; 13.05.2022 US 202217744552
(71) Applicant: Pall Corporation, Port Washington, NY 11050 (US)
(72) Inventor: CARROLL, Robin, New Port Richey, FL 34654 (US)
(74) Representative: Hoeger, Stellrecht & Partner Patentanwälte mbB

(57) **Abstract**

A filter for filtering particulates from a fluid stream includes a filter element with a filter medium and a metallic debris sensor assembly. The metallic debris sensor assembly includes a core and a coil of electrically-conductive wire. The core has a first end, a second end, and an intermediate portion interposed between the first and second ends. The first end is disposed in spaced relationship with the second end such that a measurement area is disposed therebetween. The coil of electrically-conductive wire is wound around the intermediate portion of the core. The core is adapted to generate a magnetic field in the measurement area when an electrical current is passed through the coil. At least a portion of the filter medium of the filter element is disposed within the measurement area. The filter can be incorporated into a wear detection system and used in methods of monitoring.

## Description

### CROSS-REFERENCE TO RELATED APPLICATIONS

This patent application claims the benefit of priority to U. S. Non-Provisional Patent Application No. 17/744,552, filed May 13, 2022, and entitled, "Wear Detection System with Metal Debris Sensor Assembly," which claims priority to U.S. Provisional Patent Application No. 63/276,651, filed November 7, 2021, and entitled, "Wear Detection System with Metal Debris Sensor Assembly," which are incorporated in their entirety herein by this reference.

### BACKGROUND OF THE INVENTION

Fluid used for lubricating purposes or transmitting power in mechanical systems can become contaminated by wear particles from the mechanical parts of the system over its operational time. Such particles are generated by the wear undergone by the component parts of the system, such as, bearings, gears and other metallic parts subject to frictional forces and/or stresses that cause fracture.

Lubricant condition monitoring has been used for diagnostic and prognostic assessment of the health of these mechanical parts. Evaluating debris in hydraulic, lubrication and other fluid systems can provide valuable information about abnormal contaminant ingression, accelerated part wear, impending part failure, and/or fluid break down. Monitoring of the metal particles contained in the fluid can provide early warning of the deterioration of mechanical components. The detection of such debris has been used to determine the degree of wear occurring in the system and also to predict impending failure by the presence of large or increasing amounts of debris.

Prognostics health monitoring (PHM) systems have become increasingly important to aircraft operators with respect to engine health status determination, optimizing maintenance schedules, and overall flight safety evaluation. There is a need in the art to provide additional solutions to enhance the monitoring a system of parts for wear.

It will be appreciated that this background description has been created to aid the reader, and is not to be taken as an indication that any of the indicated problems were themselves appreciated in the art. While the described principles can, in some aspects and embodiments, alleviate the problems inherent in other systems, it will be appreciated that the scope of the protected innovation is defined by the attached claims, and not by the ability of any disclosed feature to solve any specific problem noted herein.

### SUMMARY OF THE INVENTION

The present disclosure, in one aspect, is directed to embodiments of a filter. In one embodiment, a filter for filtering particulates from a fluid stream includes a filter element and a metallic debris sensor assembly. The filter element includes a filter medium. The metallic debris sensor assembly includes a core and a coil of electrically-conductive wire.

The core has a first end, a second end, and an intermediate portion interposed between the first and second ends. The first end is disposed in spaced relationship with the second end such that a measurement area is disposed therebetween. The coil of electrically-conductive wire is wound around the intermediate portion of the core. The core is adapted to generate a magnetic field in the measurement area when an electrical current is passed through the coil. At least a portion of the filter medium is disposed within the measurement area.

In another aspect, the present disclosure is directed to embodiments of a wear detection system. In one embodiment, a wear detection system for monitoring wear of a part in a system of parts includes a filter for filtering particulates from a fluid stream, a coil drive circuit, and an inductance measurement circuit.

The filter includes a filter element and a metallic debris sensor assembly. The filter element includes a filter medium. The metallic debris sensor assembly includes a core and a drive coil of electrically-conductive wire. The core has a first end, a second end, and an intermediate portion interposed between the first and second ends. The first end is disposed in spaced relationship with the second end such that a measurement area is disposed therebetween. The drive coil of electrically-conductive wire is wound around the intermediate portion of the core. The core is adapted to generate a magnetic field in the measurement area when an electrical current is passed through the drive coil. At least a portion of the filter medium is disposed within the measurement area.

The coil drive circuit is operably arranged with the drive coil and is configured to selectively provide the electrical current to the drive coil. The inductance measurement circuit is configured to determine an inductance value of the inductance measurement circuit. The inductance measurement circuit is operably arranged with the metallic debris sensor assembly such that the inductance value of the inductance measurement circuit is variable based upon an amount of magnetically responsive debris in the measurement area.

In another embodiment, an on-board wear detection system for monitoring wear of a part in a system of parts is located on a mobile machine. The on-board wear detection system includes a supply of fluid, a pump, the part, a filter for filtering particulates from the flow of fluid, a coil drive circuit, and an inductance measurement circuit.

The pump is in fluid communication with the supply of fluid. The pump is configured to draw the supply of fluid into the pump and to discharge a flow of fluid therefrom. The part is in fluid communication with the pump to receive the flow of fluid therefrom. The filter is in fluid communication with the pump and with the part. The filter is interposed between the part and the pump such that the flow of fluid passes through the filter.

The filter includes a filter element and a metallic debris sensor assembly. The filter element includes a filter medium. The metallic debris sensor assembly includes a core and a drive coil of electrically-conductive wire. The core has a first end, a second end, and an intermediate portion interposed between the first and second ends. The first end is disposed in spaced relationship with the second end such that a measurement area is disposed therebetween. The drive coil of electrically-conductive wire is wound around the intermediate portion of the core. The core is adapted to generate a magnetic field in the measurement area when an electrical current is passed through the drive coil. At least a portion of the filter medium is disposed within the measurement area.

The coil drive circuit is operably arranged with the drive coil and is configured to selectively provide the electrical current to the drive coil. The inductance measurement circuit is configured to determine an inductance value of the inductance measurement circuit. The inductance measurement circuit is operably arranged with the metallic debris sensor assembly such that the inductance value of the inductance measurement circuit is variable based upon an amount of magnetically responsive debris in the measurement area.

In yet another aspect, the present disclosure is directed to embodiments of a method of monitoring. In one embodiment, a method of monitoring for wear of a part in a system of parts includes contacting the part with a fluid such that metallic particles from the part are entrained in the fluid. The fluid is passed in a direction of flow through a filter medium of a filter element.

A metallic debris sensor assembly is operated to generate a magnetic field in a measurement area including therein an upstream portion of the filter medium relative to the direction of flow. The metallic debris sensor assembly includes a core and a drive coil of electrically-conductive wire. The core has a first end, a second end, and an intermediate portion interposed between the first and second ends. The first end is disposed in spaced relationship with the second end such that the measurement area is disposed therebetween. The drive coil of electrically-conductive wire is wound around the intermediate portion of the core. Operating the metallic debris sensor includes passing an electrical current through the drive coil of electrically-conductive wire via a coil drive circuit including the drive coil.

An inductance value of an inductance measurement circuit is determined. The inductance measurement circuit is operably arranged with the metallic debris sensor assembly such that the inductance value of the inductance measurement circuit is variable based upon an amount of magnetically responsive debris in the measurement area. A wear characteristic of the part is determined based upon the inductance value.

In another embodiment, a method of monitoring a fluid for metallic particles includes passing a fluid in a direction of flow through a filter medium of a filter element. A metallic debris sensor assembly is operated to generate a magnetic field in a measurement area including therein an upstream portion of the filter medium relative to the direction of flow. The metallic debris sensor assembly includes a core and a drive coil of electrically-conductive wire. The core has a first end, a second end, and an intermediate portion interposed between the first and second ends. The first end is disposed in spaced relationship with the second end such that the measurement area is disposed therebetween. The drive coil of electrically-conductive wire is wound around the intermediate portion of the core. Operating the metallic debris sensor includes passing an electrical current through the drive coil of electrically-conductive wire via a coil drive circuit including the drive coil.

An inductance value of an inductance measurement circuit is determined. The inductance measurement circuit is operably arranged with the metallic debris sensor assembly such that the inductance value of the inductance measurement circuit is variable based upon an amount of magnetically responsive debris in the measurement area. The amount of magnetically responsive debris in the measurement area is determined based upon the inductance value.

Further and alternative aspects and features of the disclosed principles will be appreciated from the following detailed description and the accompanying drawings. As will be appreciated, the filters, wear detection systems, and methods of monitoring disclosed herein are capable of being carried out and used in other and different embodiments, and capable of being modified in various respects. Accordingly, it is to be understood that both the foregoing general description and the following detailed description are exemplary and explanatory only and do not restrict the scope of the appended claims.

### BRIEF DESCRIPTION OF THE SEVERAL VIEWS OF THE DRAWINGS

FIG. 1 is a perspective view of an embodiment of a filter constructed in accordance with principles of the present disclosure, which includes an embodiment of a metallic debris sensor assembly constructed in accordance with principles of the present disclosure.
FIG. 2 is an exploded view of the filter of FIG. 1.
FIG. 3 is a perspective view of an embodiment of an embodiment of a metallic debris sensor assembly constructed in accordance with principles of the present disclosure.
FIG. 4 is a plan view of the metallic debris sensor assembly of FIG. 3.
FIG. 5 is a fragmentary, perspective view of a housing of the filter of FIG. 1 and the metallic debris sensor assembly of FIG. 3 installed therein.
FIG. 6 is a fragmentary, perspective view of the filter of FIG. 1, illustrating the metallic debris sensor assembly installed in the housing such that at least a portion of the filter medium of the filter is disposed within a measurement area defined by the ends of a core of the metallic debris sensor assembly and an intermediate portion of the core generally encircles the filter element.
FIG. 7 is a fragmentary, elevational view of the filter of FIG. 1 with a portion of the housing removed for illustrative purposes.
FIG. 8 is a fragmentary, elevational view of the filter of FIG. 1 with a portion of the housing removed for illustrative purposes and illustrating a fluid flow path through the measurement area defined by the core of the metallic debris sensor assembly.
FIG. 9 is a schematic view of an embodiment of a filter constructed in accordance with principles of the present disclosure, which includes an embodiment of a metallic debris sensor assembly constructed in accordance with principles of the present disclosure connected to a power source.
FIG. 10 is a perspective view of an embodiment of a core for a metallic debris sensor assembly constructed in accordance with principles of the present disclosure.
FIG. 11 is a plan view of the core of FIG. 10.
FIGS. 12 and 13 are perspective views of a filter constructed in accordance with principles of the present disclosure, with a portion of the housing removed for illustrative purposes and illustrating the relative placement of the core of FIG. 3 and the core of FIG. 10.
FIG. 14 is a schematic view of an embodiment of a metallic debris sensor assembly constructed in accordance with principles of the present disclosure which includes a coil drive circuit with a drive coil and and an inductance measurement circuit with a pickup coil.
FIG. 15 is a schematic view of an embodiment of a metallic debris sensor assembly constructed in accordance with principles of the present disclosure which includes a coil drive circuit with a drive coil and an inductance measurement circuit with a pair of pickup coils.
FIG. 16 is a schematic view of an embodiment of a metallic debris sensor assembly constructed in accordance with principles of the present disclosure which includes a coil drive circuit and an inductance measurement circuit with a common coil and an inductance measurement reference circuit.
FIG. 17 is a schematic view of an embodiment of a metallic debris sensor assembly constructed in accordance with principles of the present disclosure which includes a coil drive circuit with a drive coil, an inductance measurement circuit with a pickup coil, and an inductance measurement reference circuit.
FIG. 18 is a schematic view of an embodiment of a metallic debris sensor assembly constructed in accordance with principles of the present disclosure which includes a coil drive circuit with a drive coil, an inductance measurement circuit with a pair of pickup coils, and an inductance measurement reference circuit.
FIG. 19 is a schematic view of an embodiment of an onboard wear detection system including an embodiment of a metallic debris sensor system constructed in accordance with principles of the present disclosure.

It should be understood that the drawings are not necessarily to scale and that the disclosed embodiments are illustrated diagrammatically and in partial views. In certain instances, details which are not necessary for an understanding of this disclosure or which render other details difficult to perceive may have been omitted. It should be understood that this disclosure is not limited to the particular embodiments illustrated herein.

### DETAILED DESCRIPTION OF THE INVENTION

Embodiments of a filter constructed in accordance with principles of the present disclosure are adapted to be used with embodiments of a wear detection system constructed in accordance with principles of the present disclosure for detecting wear of a part in a fluidic system. Embodiments of a filter and a wear detection system constructed in accordance with principles of the present disclosure can be used in embodiments of a method of monitoring a fluid following principles of the present disclosure.

In embodiments, a filter constructed in accordance with principles of the present disclosure includes a filter element, a metallic debris sensor assembly, and a housing. In embodiments, a filter element includes a filter medium configured to capture metallic debris particles generated within a system. The metallic debris sensor assembly can be fitted in such a way that the metallic debris collected within the measurement area of the filter media 29 provides an inductance change to an inductance measurement circuit associated with the metallic debris sensor assembly. This induction change can be tracked over time to determine cumulative wear debris collected within the measurement area. Debris within the measurement area can also be probed via frequency analysis techniques to determine the type(s) of debris materials within the measurement area.

In embodiments, a wear detection system includes a filter, a coil drive circuit, and an inductance measurement circuit. The filter includes a filter element and a metallic debris sensor assembly. The metallic debris sensor assembly can include a core and an electrically-conductive wire coiled around the core such that, when an electric current is passed through the wire via the coil drive circuit, a magnetic field is generated via the core, including within the measurement area. The core is of such a geometry that it has a gap in its structure which delimits the measurement area. The metallic debris sensor assembly can be arranged with the filter element such that at least a portion of the filter medium is disposed in the measurement area. When the filter medium within the measurement area collects metallic debris, the inductance measured by the inductance measurement circuit changes proportionally to the mass of magnetically-responsive debris collected in the measurement area.

In embodiments of a wear detection system constructed in accordance with principles of the present disclosure, the coil of electrically-conductive wire is connected to a coil drive circuit. In embodiments, the coil drive circuit is connected to an external power source. In embodiments, an inductance measurement circuit comprises an inductance measurement system configured to measure the value of the inductance of the inductance measurement circuit. In embodiments, the coil drive circuit and the inductance measurement circuit comprise the same circuit which performs both functions.

In embodiments, the metallic debris sensor assembly can comprise a coil, which is a drive coil, and at least one secondary pickup coil to enhance the sensitivity of the metallic debris sensor assembly. The drive coil is connected to the external power source via the coil drive circuit, and the secondary pickup coil is connected to the inductance measurement system.

In embodiments, an inductance signal is transmitted from the inductance measurement circuit to a processor programmed with a sensor debris detection program stored upon a non-transitory computer-readable medium to generate a wear characteristic and/or determine the amount of magnetically responsive debris accumulated in the measurement area. In embodiments, the sensor debris detection program is configured to determining a debris generation rate. In embodiments, the wear characteristic and/or debris generation rate can be compared to predetermined threshold levels and the processor can be configured to issue a warning signal or maintenance alert via a user interface device. The sensor debris detection program can be configured to conduct a debris material frequency response analysis to identify at least one part of the system of parts generating magnetically responsive debris and/or to identify the magnetically responsive debris. In embodiments of a wear detection system constructed in accordance with principles of the present disclosure, the wear detection system can include one or more filters constructed in accordance with principles of the present disclosure, such as one in a position upstream of a part being monitored and another in a position downstream of the part.

Embodiments of a filter constructed in accordance with principles of the present disclosure can be used in mobile machine environments, such as in an onboard wear detection system constructed in accordance with principles of the present disclosure and installed in a mobile machine, such as an aircraft, for example, but can be used in other industrial applications where system wear monitoring is desired. Embodiments of a filter constructed in accordance with principles of the present disclosure can be used to filter particulates from a fluid stream used in hydraulic or lubricant applications that are prone to generate metallic debris over time based upon the wear of one or more parts. Embodiments of a filter constructed in accordance with principle of the present disclosure can be used to monitor a part over its maintenance life.

It will be appreciated that this detailed description provides exemplary embodiments of the invention. Since other embodiments of the invention may differ in detail from the embodiments in this detailed description, the detailed description is intended to reference the particular embodiments being discussed at that point and is not intended to imply any limitation as to the scope of the invention more generally.

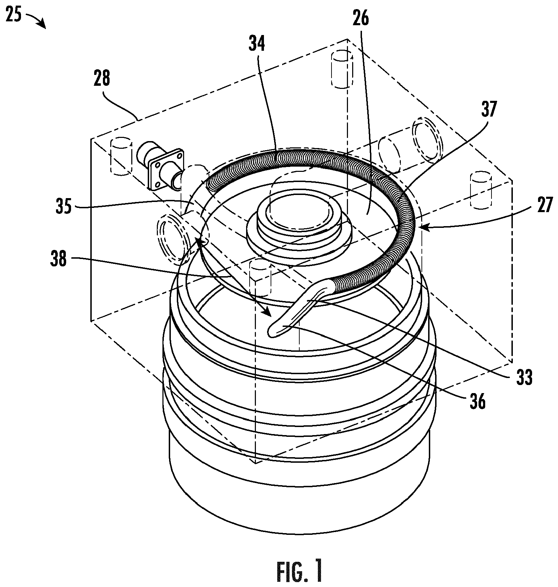

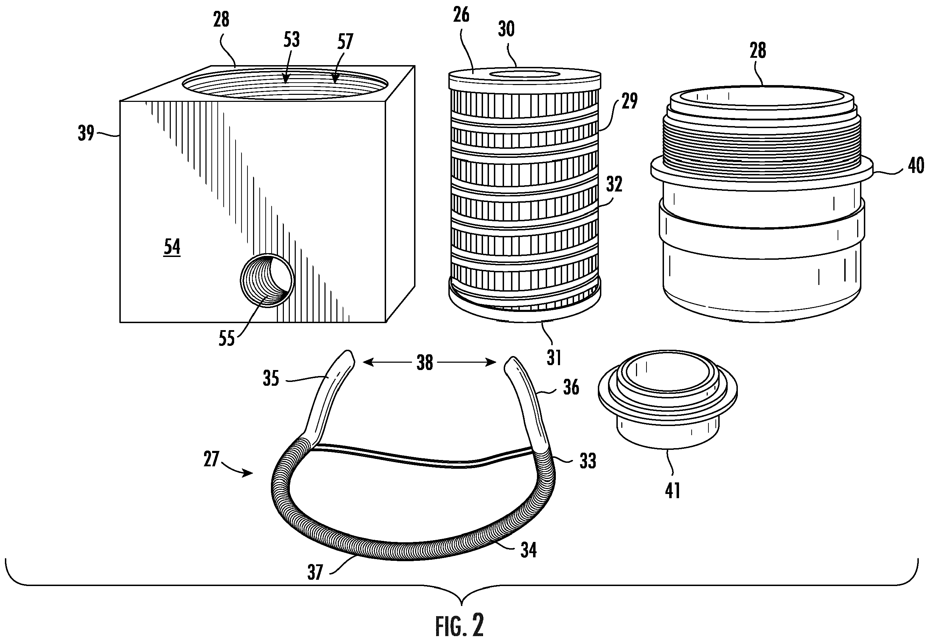

Turning now to the FIGURES, there is shown in FIGS. 1 and 2 an embodiment of a filter 25 constructed in accordance with principles of the present disclosure that includes a filter element 26. In the illustrated embodiment, the filter 25 includes a filter element 26, a metallic debris sensor assembly 27 constructed in accordance with principles of the present disclosure, and a housing 28. The filter 25 is configured to filter particulates from a fluid stream or flow.

Referring to FIG. 2, the filter element 26 (also referred to as a "cylindrical pack") is cylindrical and is adapted to filter material in response to fluid flow therethrough. The illustrated filter element 26 includes a filter medium 29, a first end cap 30, a second end cap 31, and a helical wrap 32.

The filter element 26 extends along a longitudinal axis between a first axial end and a second axial end. The filter element 26 has an inner surface defining an interior passage. An outer surface of the filter element 26 defines an outer cylindrical surface area. One skilled in the art will appreciate that in other embodiments, the filter element 26 can have a different shape, including non-cylindrical shapes having a cross-section with an outer periphery that is non-circular, such as, oval, square, triangular, hexagonal, for example.

The first and second end caps 30, 31 (also referred to as "adapters") are respectively secured to the first axial end and the second axial end of the filter element 26 to form a fluid tight seal therebetween. The end caps 30, 31 respectively seal the ends of the filter element 26. In embodiments, at least one of the first end cap 30 and the second end cap 31 defines an opening therethrough in fluid communication with the interior passage of the filter element 26 (see FIG. 2). The wrap 32 is helically wrapped around an outer surface of the filter element 26 and can be configured to help maintain the structural shape of the filter element 26 during use, particularly in applications in which the direction of fluid flow is from the interior passage out to the exterior of the cylindrical pack. In other embodiments, the wrap 32 can be omitted.

In embodiments of a filter 25 constructed according to principles of the present disclosure, the filter element 26 is equipped with end caps 30, 31 at one or both of its axial ends. The end caps 30, 31 can be blind or open end caps, and can be made of a material which is suitable for the filter conditions and the other materials of the filter 25 components to which the end caps 30, 31 are to be joined. In embodiments, the end caps 30, 31 are attached to the filter element 26. Conventional techniques can be used to attach the end caps 30, 31 to the filter element 26, such as, by polycapping or spin welding, or by using an epoxy, for example.

In embodiments, the filter medium 29 is configured to filter particulates from a fluid stream. In embodiments, any suitable filter medium 29 can be employed in a filter 25 constructed according to principles of the present disclosure, and it can be selected in accordance with the fluid which is intended to be filtered and the desired filtering characteristics. The filter medium 29 can be used to filter a variety of fluid streams, such as, lubricant, hydraulic oil, liquid fuel, etc.

In embodiments, the filter medium 29 can comprise a membrane, a porous film or a fibrous sheet or mass; it may have a uniform or graded pore structure and any appropriate effective pore structure, e.g., pore size, pore rating, or pore diameter. The filter medium 29 can be formed from any suitable material, such as a natural or synthetic polymer or glass. The filter medium 29 may comprise a single layer, or the filter medium 29 may comprise a plurality of layers of the same medium disposed atop one another to a desired thickness. Furthermore, it is possible for the filter medium 29 to include two or more layers having different filtering characteristics, e.g., with one layer acting as a prefilter for the second layer. In embodiments, the filter element 26 comprises several integral regions, including a single, unitary porous sheet having a finely-pored center region, which serves as a filter medium 29, and coarsely-pored upstream and/or downstream regions which serve as drainage layers.

Meshes are particularly suitable as drainage layers when the filter medium 29 is a fibrous laid-down medium. On the other hand, when the filter medium 29 is a membrane, a woven or non-woven fabric may be more suitable for use as the drainage layer(s) because a fabric is usually smoother than a mesh and produces less abrasion of adjoining layers of the filter composite.

In embodiments, the filter element 26 can include a cylindrical core which is coaxially disposed within the interior passage and which is familiar to one skilled in the art. The core defines a plurality of openings disposed in spaced relationship to each other in a regular pattern to permit the passage of a fluid stream either radially outward from an axial central bore of the core or radially inward from the outside of the core to the central bore. In embodiments, the core can have any suitable construction, including being of conventional design and being made of any material having sufficient strength for the intended application and being compatible with the fluid intended to be filtered, as will be appreciated by one skilled in the art.

Referring to FIG. 1, the metallic debris sensor assembly 27 includes a core 33 and a coil of electrically-conductive wire 34. The core 33 has a first end 35, a second end 36, and an intermediate portion 37 interposed between the first and second ends 35, 36. It should be understood that designations such as "first" and "second" are for convenient reference only and are not limiting and the designation of a particular part can be changed without altering its structure or operation. The first end 35 is disposed in spaced relationship with the second end 36, such that a measurement area 38 is disposed therebetween. The measurement area 38 is an open area defined between the first end 35 and the second end 36. The core 33 is arranged with the filter element 26 such that the filter medium 29 of the filter element 26 is disposed within the measurement area 38. The coil of electrically-conductive wire 34 is wound around the intermediate portion 37 of the core 33. The core 33 is adapted to generate a magnetic field in the measurement area 38 when an electrical current is passed through the coil 34. In embodiments, the measurement area 38 can be positioned within at least a portion of the filter medium 29 configured to capture magnetically susceptible particles of interest within the measurement area 38. In the illustrated embodiment, the coil 34 is a drive coil and is used by an inductance measurement system to determine the inductance.

The core 33 can be any suitable shape according to the intended application in which it defines a measurement area 38 between the first end 35 and second end 36. In embodiments, the core 33 is generally C-shaped. In embodiments, the core 33 can be a curved "C" or a block "C." In embodiments, the core 33 is of a shape such that it may be installed in a filter 25 as described herein. In embodiments, the core 33 is installed by any suitable means to the particular filter.

The intermediate portion 37 can be any suitable shape according to the intended application. Suitable shapes include a toroidal shape. In embodiments, the intermediate portion 37 is a toroidal segment. In embodiments, the cross section or diameter of the core 33 is substantially constant over its length. In embodiments, the core 33 is substantially constant over its length until tapering at the ends 35, 36. In embodiments, the first and second ends 35, 36 can be tapered in a way such that the measurement area 38 is the shortest distance between the core's first and second ends 35, 36.

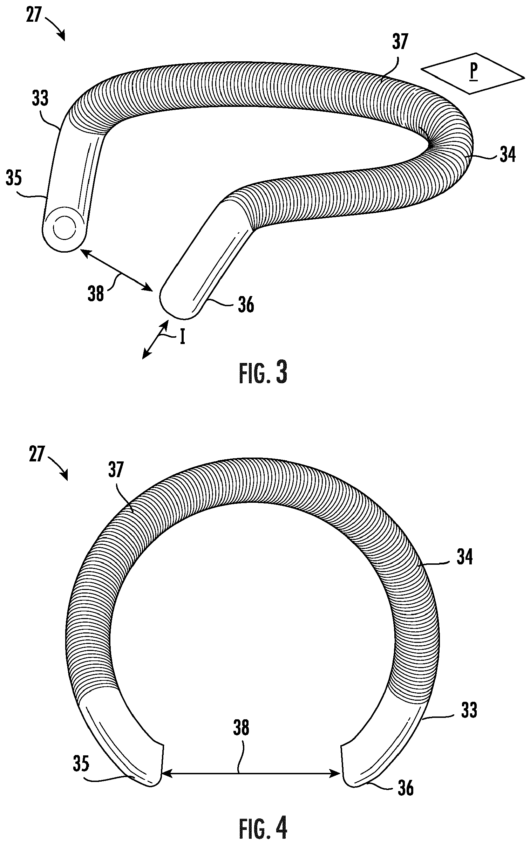

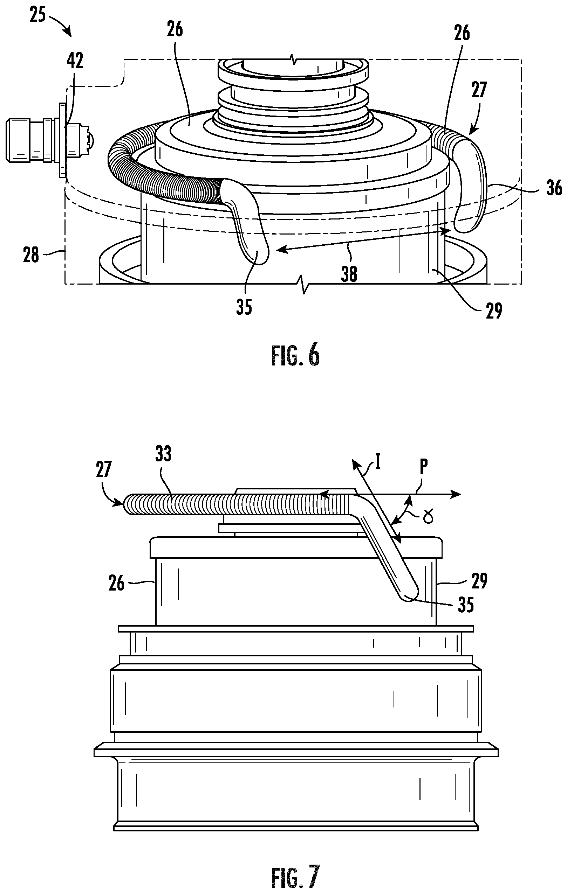

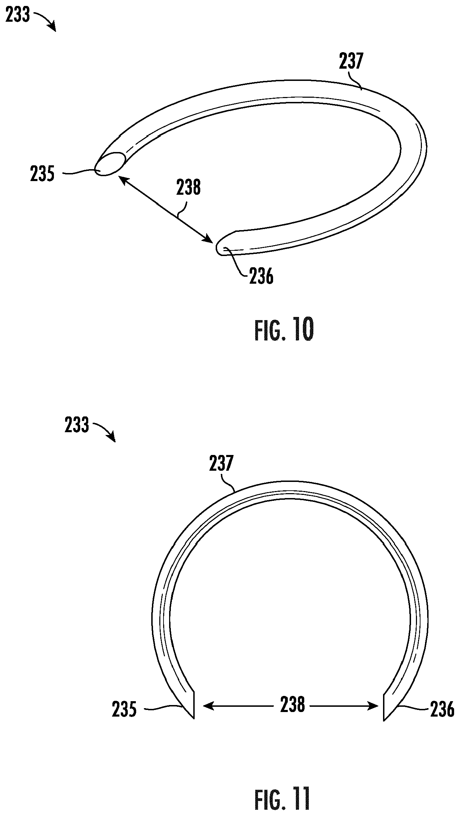

In embodiments, the intermediate portion of the core 37 is disposed along a principal core plane P, and the first 35 and second ends 36 extend from the intermediate portion 37 along a respective inclined axis I, the inclined axis I being in non-parallel relationship with the principal core plane P *(see, e.g.,* FIGS. 3 and 7). In embodiments, the principal core plane P and the inclined axis I define a respective acute angle γ therebetween. In embodiments, the acute angle γ is greater than five degrees (e.g., greater than five degrees, greater than 15 degrees, greater than 25 degrees, greater than 35 degrees, greater than 45 degrees, greater than 55 degrees, greater than 65 degrees, greater than 75 degrees, or greater than 85 degrees). In embodiments, the acute angle γ is less than 90 degrees (e.g., the acute angle is less than 90 degrees, less than 80 degrees, less than 70 degrees, less than 60 degrees, less than 50 degrees, less than 40 degrees, less than 30 degrees, less than 20 degrees, or less than 10 degrees). In embodiments, the acute angle γ is greater than five degrees and less than ninety degrees. In other embodiments, the first 235 and second ends 236 and the intermediate portion 237 are substantially coplanar, as shown in FIGS. 10 and 11.

The core 33 may comprise any suitable material for the intended purpose as described herein. For example, the core 33 may comprise any material that has suitable electrical conductivity, magnetic permeability, and coercivity to detect metallic debris according to the intended purpose. The core 33 may also comprise a material that has sufficient magnetic susceptibility, such that it amplifies the magnetic field within the measurement area 38 over that of the coil of electrically-conductive metal alone and provides a sufficient magnetic flux density within the measurement area 38 for its intended application.

In embodiments, the core 33 comprises a soft magnet, such as ferrite. In embodiments, the core 33 comprises a material with suitable magnetic remanence (i.e., it remains highly magnetized in absence of an external field). In embodiments, the core 33 comprises at least one of: a magnet ferrite, a ferromagnetic amorphous metal alloy, a nickel-iron alloy, an electrical steel, a powdered metal, a ferritic stainless steel, and a martensitic stainless steel. In embodiments, the core 33 comprises at least two, at least three, at least four, at least five, at least six, or all seven of: a magnet ferrite, a ferromagnetic amorphous metal alloy, a nickel-iron alloy, an electrical steel, a powdered metal, a ferritic stainless steel, and a martensitic stainless steel. In embodiments, the core 33 comprises at least one of a manganese-zinc ferrite and a nickel-zinc ferrite. In embodiments, the core 33 comprises a manganese-zinc ferrite or a nickel-zinc ferrite. In embodiments, the core 33 comprises a manganese-zinc ferrite and a nickel-zinc ferrite. In embodiments, the core 33 comprises a ferromagnetic amorphous metal alloy including: (i) at least one of boron, silicon, and phosphorous and (ii) at least one of iron, nickel, and cobalt. In embodiments, the core 33 comprises a ferromagnetic amorphous metal alloy including: (i) at least two of boron, silicon, and phosphorous and (ii) at least two of iron, nickel and cobalt. In embodiments, the core 33 comprises a ferromagnetic amorphous metal alloy including: (i) boron, silicon, and phosphorous and (ii) iron, nickel, and cobalt.

In embodiments, the core 33 comprises an iron alloy with up to 6.5 wt.% silicon, up to 0.5 wt.% manganese, and up to 0.5 wt.% aluminum. For example, in some embodiments the core 33 comprises an iron alloy with up to 6.5 wt.% silicon (e.g., up to 0.5 wt.%, up to 1.0 wt.%, up to 1.5 wt.%, up to 2.0 wt.%, up to 2.5 wt.%, up to 3.0 wt.%, up to 3.5 wt.%, up to 4.0 wt.%, up to 4.5 wt.%, up to 5.0 wt.%, up to 5.5 wt.%, up to 6.0 wt.%, or up to 6.5 wt.%). In embodiments, the core 33 comprises an iron alloy with up to 0.5 wt.% manganese (e.g., up to 0.1 wt.% , up to 0.2 wt.%, up to 0.3 wt.%, up to 0.4 wt.%, or up to 0.5 wt.%,). In embodiments, the core 33 comprises an iron alloy with up to 0.5 wt.% aluminum (e.g., up to 0.1 wt.% , up to 0.2 wt.%, up to 0.3 wt.%, up to 0.4 wt.%, or up to 0.5 wt.%).

In embodiments, the core 33 comprises at least one of a powdered iron, a powdered carbonyl iron, and a powdered hydrogen-reduced iron. In embodiments, the core 33 comprises at least two of a powdered iron, a powdered carbonyl iron, and a powdered hydrogen-reduced iron. In embodiments, the core 33 comprises a powdered iron, a powdered carbonyl iron, and a powdered hydrogen-reduced iron.

In embodiments, the core 33 may be a complex shape and made of stainless steel, which can be shaped by any suitable means known to a person skilled in the art, such as a mill or lathe. In other embodiments, the core 33 may be a simple shape and made of ferrite ceramic, which can be shaped by any suitable means known to a person skilled in the art, such as sintering and grinding.

In embodiments, the coil of electrically-conductive wire 34 is wound around the intermediate portion of the core 37. The core 33 is adapted to generate a magnetic field in the measurement area 38 when an electrical current is passed through the coil. In embodiments, the wire 34 is wrapped around a portion of the core 33. Any suitable electrically-conductive wire 34 can be used for the coil for the purpose of creating a magnetic field according to principles described herein, as will be appreciated by a person skilled in the art.

In embodiments, the coil 34 is a tightly wrapped wire coil around the core 33 material in order to induce a magnetic flux density within the core 33. In embodiments, each end of the coiled wire 34 has a sufficient length to connect to a coil drive circuit. In embodiments, the coil drive circuit is connected to a power supply and an inductance measurement system. Any suitable power supply that is capable of controlling input power and frequency of the electrical current being applied can be used in the metallic debris sensor assembly. In embodiments, the power supply can comprise an AC power supply. In other embodiments, the power supply can comprise a DC power supply in combination with a function generator or DC-AC converter which is then used to power the sensor coil 34. Any suitable inductance measurement system can be used in the metallic debris sensor assembly described herein.

In embodiments, the core 33 is wrapped in multiple (i.e., more than one, more than two or more than three) layers of electrically-conductive wire in wire coils. In embodiments, the multiple layers of electrically-conductive wire provides a stronger magnetic field or higher magnetic sensitivity than a single (i.e., one) layer.

Referring to FIG. 1, in embodiments, the housing 28 can be provided for storing at least a portion of the filter element 26 and the metallic debris sensor assembly 27 therein. The housing 28 can be constructed from any suitable material, such as, aluminum, a non-metallic material, or a non-magnetically-response material, for example.

Referring to FIG. 2, in embodiments, the housing 28 includes an interior surface 53 and an exterior surface 54 and defines a through passage 55 between the exterior surface 54 and the interior surface 53. The interior surface 53 defines a cavity 57 for housing the filter element 26 therein. In embodiments, the filter 25 and the core 33 are each disposed at least partially in the cavity 57 of the housing 28.

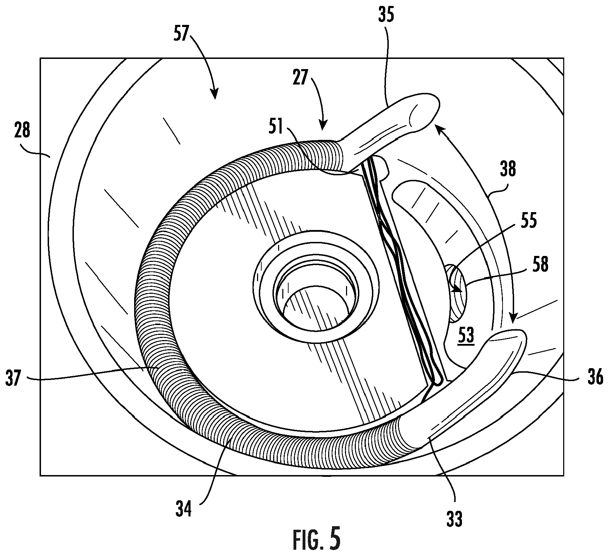

As shown in FIG. 5, the through passage 55 includes an inner opening 58 which is defined by the interior surface 53 and is in fluid communication with the cavity 57. The inner opening 58 of the through passage 55 is disposed between the first end 35 and the second end 36 of the core 33 such that the measurement area 38 is substantially axially aligned with the inner opening 58.

In embodiments, the housing 28 comprises a metal, and the first end 35 and the second end 36 of the core 33 are disposed in spaced relationship to the interior surface 53 of the housing 28. In embodiments, the metallic debris sensor assembly 27 is configured to fit in the housing 28 and is of such a geometry that it has minimal impact on the fluid flow path and a measurement area 38 through a cross section of the filter media 29 such that the centerline orthogonal to the cross section passes through the filter media 29 while avoiding metallic components of the filter element 26 such as the filter core, endcap, and housing.

Referring to FIG. 2, embodiments of separate components of a filter 25 constructed in accordance with principles of the present disclosure are shown. FIG. 2 shows the filter element 26, the metallic debris sensor 27, and the housing 28. In the illustrated embodiment shown in FIG. 2, the housing 28 includes a head 39 and a body 40 which can be connected together to comprise the housing 28. In embodiments, the housing 28 can have a different configuration and/or be comprised of a single components or more than two components.

FIG. 2 also shows an element adapter 41. The element adapter 41 is configured to facilitate the fluid connection of the filter 25 to a supply of fluid such that the fluid is in communication with the interior of the filter element 26.

FIGS. 3 and 4 depict the metallic debris sensor assembly 27 from two different vantage points. The metallic debris sensor assembly 27 includes the core 33 and the coil of electrically-conductive wire 34 as described herein.

Referring to FIG. 5, an embodiment of a metallic debris sensor assembly 27 is shown disposed inside of a groove 51 defined by the interior of the housing 28. In embodiments, the groove 51 can be of a shape and size to permit the intermediate portion 37 of the core 33 to be disposed within the groove and the first 35 and second ends 36 of the core 33 to project out from the groove into the cavity. In embodiments, the intermediate portion 37 of the core 33 can be secured to the housing 28 by filling the groove 51 with a suitable potting compound, as will be known to one skilled in the art. In embodiments, potting compound is disposed in the groove 51 such that the intermediate portion 37 of the core 33 is embedded in the potting compound. Exemplary suitable potting compounds include an epoxy, a polyurethane, or other adhesive known to be suitable by a person skilled in the art. Any suitable potting compound can be used in accordance with the intended purpose of disposing or adhesively bonding the intermediate portion 37 of the core 33 to the housing. In other embodiments, thte core 33 can be attached to the housing via a suitable mechanical connection as will be appreciated by one skilled in the art.

Referring to FIG. 6, a filter 25 constructed in accordance with the principles of the present disclosure is shown. The metallic debris sensor assembly 27 is installed in the housing 28 such that at least a portion of the filter medium 29 of the filter 25 is disposed within the measurement area 38 defined by the space between the first end 35 and second end 36 of the core 33.

FIG. 6 also shows a sensor port 42 defined by the housing 28 and extending between the exterior surface and the interior surface of the housing 28 to provide access to the metallic debris sensor assembly disposed within the cavity of the housing 28. In embodiments, the sensor port 42 can be used to associate the metallic debris sensor assembly 27 with the coil drive circuit and the inductance measurement circuit. In embodiments, the housing 28 can define one or more sensor ports having different configurations so as to facilitate the electrical connection of the metallic debris sensor assembly 27 to an external power source and an inductance measurement system. Referring to FIGS. 6 and 7, the metallic debris sensor assembly 27 is shown installed, such that at least a portion of the filter medium 29 of the filter 26 is disposed within the measurement area 38 defined between the first end 35 and second end 36 of the core 33.

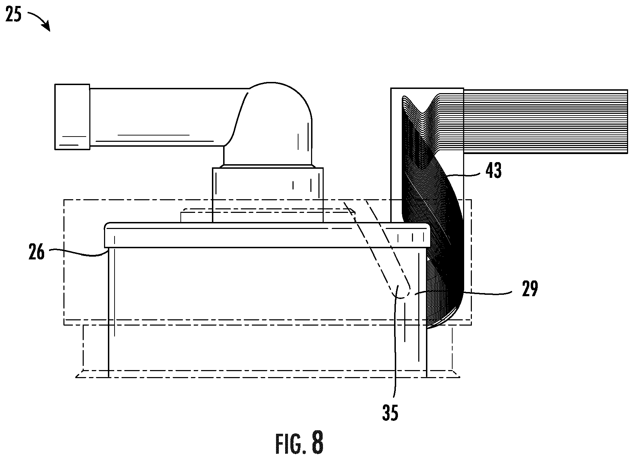

FIG. 8 shows an exemplary fluid flow path 43 moving through the filter element 26. I embodiments, fluid can pass through a portion of the filter medium 29 of the filter 25 that is disposed within the measurement area 38 defined by the space between the first end 35 and second end 36 of the core 33.

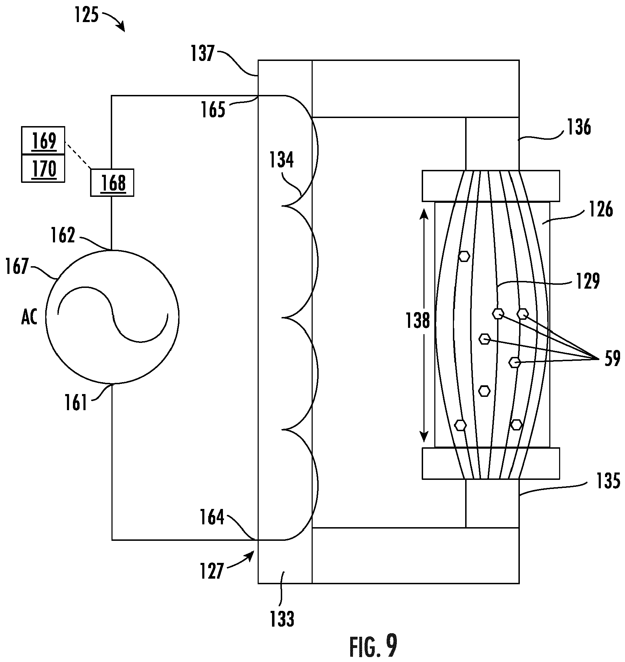

Referring to FIG. 9, a schematic view of a filter 125 constructed according to principles of the present disclosure is shown which includes a filter element 126 and a metallic debris sensor assembly 127 constructed in accordance with principles of the present disclosure.

The metallic debris sensor assembly 127 includes a core 133 and a coil of electrically-conductive wire 134. The core 133 has a first end 135, a second end 136, and an intermediate portion 137 interposed between the first and second ends 135, 136. The first end 135 is disposed in spaced relationship with the second end 136, such that a measurement area 138 is disposed therebetween. The core 133 is arranged with the filter element 126 such that the filter medium 129 of the filter element 126 is disposed within the measurement area 138. The coil of electrically-conductive wire 134 is wound around the intermediate portion 37 of the core 133.

Fluid containing metallic debris particles 59 (also described as metal particulates or metallic particles or magnetically responsive debris) passes in a direction of flow through the filter medium 129 of the filter element 126. At least a portion of the filter element 126 is disposed within the measurement area 138 between the first end 135 and the second end 136 of the core 133.

In embodiments, the metallic debris sensor assembly 127 utilizes the coiled wire 134 as both an input and output source. In embodiments, this coil 134 is a drive coil. In embodiments, the drive circuit 161 and the inductance measurement circuit 162 both include the drive coil 134, and the inductance value is determined using the drive coil 134. In the illustrated embodiment, the ends 164, 165 of the coil 134 of the electrically-conductive wire are attached to the drive circuit 161 and the inductance measurement circuit 162 for connecting the metallic debris sensor assembly 127 to an external power source 167 and an inductance measurement system 168. In embodiments, the input is an electrical current of controlled voltage and frequency. In embodiments, the output is the inductance value of an inductance measurement circuit.

When an electric current is passed through the electrically-conductive wire 134, a magnetic field is induced in the core 133 and the measurement area 138. In embodiments, the electric current is a specific power voltage or frequency or combination of power voltage or frequency. The filter medium 129 within the measurement area 138 (i.e., between the first end 135 and the second end 136 of the core 133) can collect metallic debris 59. In embodiments, the metallic debris 59 is debris of a ferro/ferri/paramagnetic/diamagnetic or electrically conductive nature. When metallic debris 59 is collected within the measurement area 138, an inductance change will occur. The inductance measurement system 168 can measure the inductance value of an inductance measurement circuit. The inductance can change proportionally to the mass of the magnetically-responsive debris 59 collected in the sensing area.

In embodiments, the inductance measurement system 168 is configured to transmit an inductance signal to a processor 169 programmed with a sensor debris detection program contained on a non-transitory computer readable medium 170. In embodiments, the inductance signal corresponds to the inductance value of the inductance measurement circuit 162. Any suitable processor for the purposes described herein can be used as will be appreciated by one skilled in the art.

In embodiments, the power source 167 is sufficient to overcome the core material energy losses (e.g., eddy currents, magnetization energy storage) and to produce a magnetic field over the measurement area 138. In embodiments, a power threshold can exist unique to the material and geometry where the debris inductance value can be overshadowed by the existing field strength (i.e. the field is so strong that a small amount of debris does not significantly change the inductance value of the inductance measurement circuit). Within the described power range, typically an increase in power results in a higher magnetic field strength in the measurement area 138. In embodiments, the power source 167 is provided that operates with at least a portion of the described power range.

In embodiments, the magnetic field magnitude within the measurement area 138 is increased and allowed to remain in one polarization direction (i.e., DC field) and is used as a capture device for metallic debris particles. In embodiments, the magnetic field can be kept constant to magnetically capture debris particles and measure change in the inductance value of an inductance measurement circuit to detect debris.

The sensor debris detection program can be configured to track the inductance value over time such that the inductance change over time at a specific input can be compared. In embodiments, the inductance value is determined at least periodically by an inductance measurement system. For example, in embodiments, an inductance measurement is recorded with a clean filter media 129 in the measurement area 138. Then metallic debris 59, for example, iron particles, are introduced to the measurement area 138 and another inductance measurement is recorded. The change in those measurements can be correlated to an amount of magnetically responsive debris that has accumulated in the time between measurements.

FIGS. 10 and 11 show two views of another embodiment of a core 233 which is suitable for use in embodiments of a metallic debris sensor assembly constructed according to principles of the present disclosure. The core 233 of FIGS. 10 and 11 includes a first end 235 and a second end 236 that are substantially coplanar with an intermediate portion 237 of the core 133.

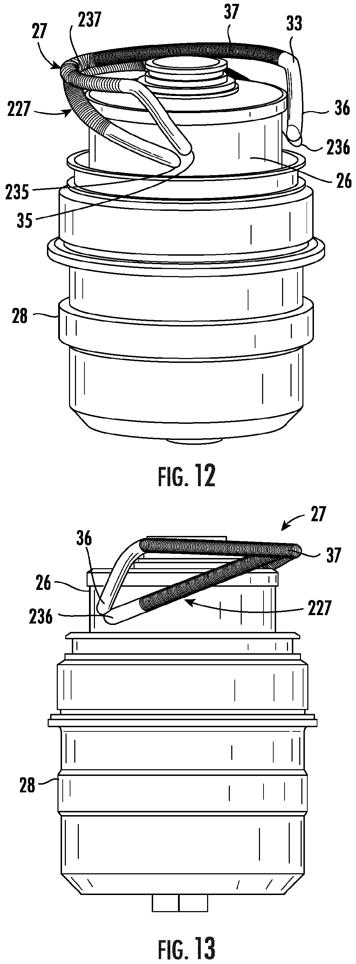

FIGS. 12 and 13 show comparative views of the relative placement of a pair of metallic debris sensor assemblies 27, 227 which respectively include the core 33 of FIG. 3 and the core 233 of FIG. 10. In the embodiment of the metallic debris sensor assembly 227 that includes the core 233 of FIG. 10, the intermediate portion 237 of the core 233 is substantially coplanar with the first end 235 and the second end 236 of the core 233. It should be understood that, in use, one metallic debris sensor assembly is provided. FIGS. 12 and 13 simply demonstrate the relative differences in the configuration and position of the metallic debris sensor assemblies 27, 227 when installed in the housing 28 and arranged with the filter element 26.

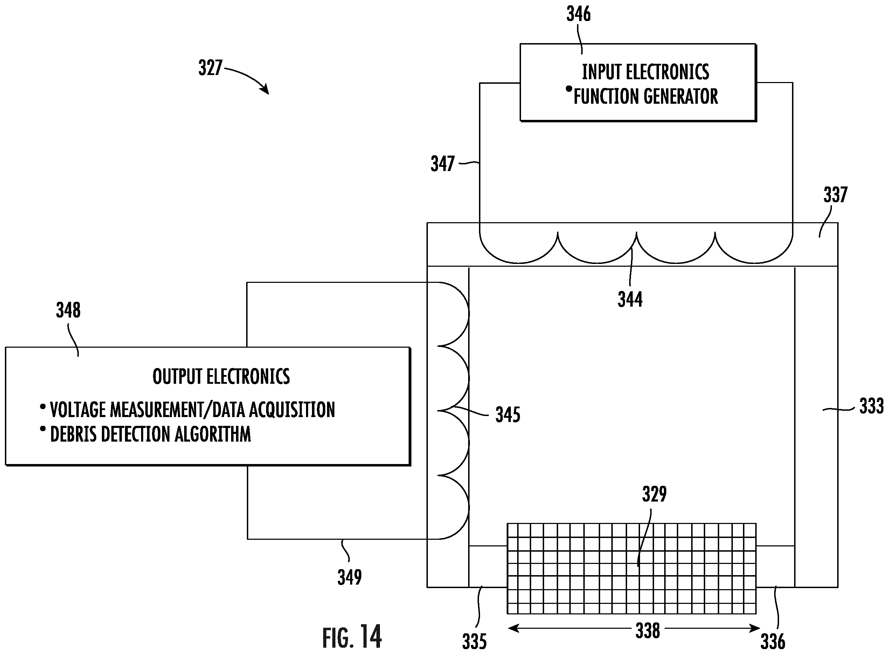

FIG. 14 shows a schematic view of an embodiment of a metallic debris sensor assembly 327 constructed in accordance with principles of the present disclosure which includes a drive coil 344 and a pickup coil 345 of electrically-conductive wire. The pickup coil 345 is wound around the intermediate portion 337 of the core 333. In embodiments, the drive coil 344 is in substantial non-overlapping relationship with the pickup coil 345 along the intermediate portion 337 of the core 333. In embodiments, the drive coil 344 (the power coil or input coil) is connected to an external power source 346 via a suitable coil drive circuit 347 (the power circuit or input circuit).

In embodiments, the inductance value is determined using the pickup coil 345 to enhance the sensitivity of the metallic debris sensor assembly 327 as compared to a metallic debris sensor assembly comprising a single coil in both the coil drive circuit and the inductance measurement circuit. In embodiments, the pickup coil 345 (a passive coil or output coil) is connected to an inductance measurement system 348 via an inductance measurement circuit 349 which does not include the drive coil 344. In embodiments, the inductance value of the inductance measurement circuit 349 is variable based on an amount of current induced in the pickup coil 345 based upon the amount of magnetically responsive debris in the measurement area 338 between the first and second ends 335, 336 of the core 333.

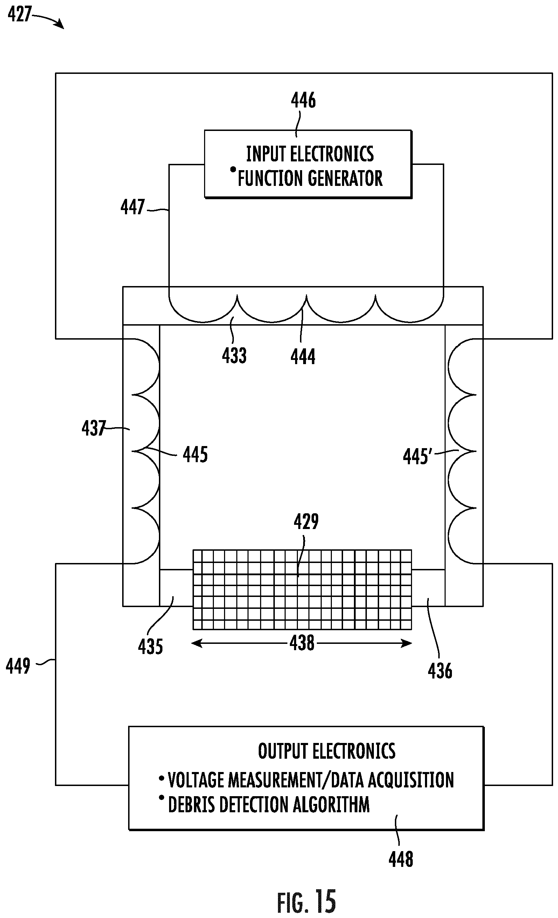

FIG. 15 shows a schematic view of an embodiment of a metallic debris sensor assembly 427 constructed in accordance with principles of the present disclosure which includes a drive coil 444, a first pickup coil 445 of electrically-conductive wire, and a second pickup coil 445' of electrically-conductive wire. The first pickup coil 445 and the second pickup coil 445' are both wound around the intermediate portion 437 of the core 433 as shown in FIG. 15. The drive coil 444 is in substantial non-overlapping relationship with both the first pickup coil 445 and the second pickup coil 445' along the intermediate portion 437. In embodiments, the drive coil 444 is connected to the external power source 446 via a coil drive circuit 447.

In embodiments, the first pickup coil 445 and the second pickup coil 445' are both connected to an inductance measurement system 448 via an inductance measurement circuit 449. In embodiments, the inductance measurement circuit 449 includes the first pickup coil 445 and the second pickup coil 445'. The inductance value of the inductance measurement circuit 449 is variable based upon at least one of a first amount of current induced in the first pickup coil 445 based on the amount of magnetically responsive debris in the measurement area 438 and a second amount of voltage inducted in the second pickup coil 445' based upon the amount of magnetically responsive debris in the measurement area 438. The inductance value can be determined using at least one of the first pickup coil 445 and the second pickup coil 445'.

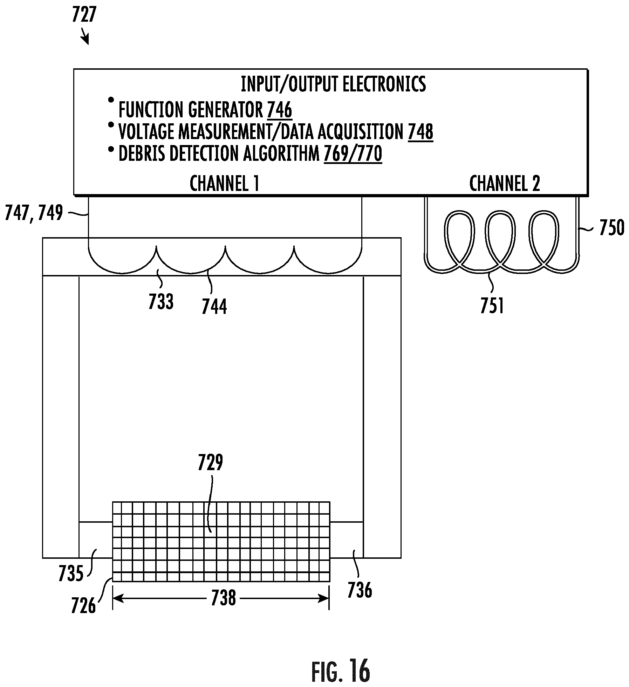

FIG. 16 shows a schematic view of another embodiment of a metallic debris sensor assembly 727 constructed in accordance with principles of the present disclosure which includes a coil drive circuit 747 and an inductance measurement circuit 749 with a common coil 744 and an inductance measurement reference circuit 750 with a reference coil 751.

The metallic debris sensor assembly 727 includes a core 733 and the common coil of electrically-conductive wire 744. The core 733 is arranged with the filter element 726 such that the filter medium 729 of the filter element 726 is disposed within the measurement area 738. The coil of electrically-conductive wire 734 is wound around the intermediate portion 737 of the core 733.

Fluid containing metallic debris particles (also described as metal particulates or metallic particles or magnetically responsive debris) passes in a direction of flow through the filter medium 729 of the filter element 726. At least a portion of the filter element 726 is disposed within the measurement area 738 between the first end 735 and the second end 736 of the core 733.

In embodiments, the metallic debris sensor assembly 727 utilizes the common coil 744 as both an input and output source. In embodiments, this coil 744 is a drive coil. In embodiments, the coil drive circuit 747 and the inductance measurement circuit 749 both include the drive coil 744, and the inductance value is determined using the drive coil 744 with reference to the inductance measurement reference circuit 750. In the illustrated embodiment, the common coil 744 of the electrically-conductive wire is attached to the coil drive circuit 747 and the inductance measurement circuit 749 for connecting the metallic debris sensor assembly 727 to a power source 746 and an inductance measurement system 748 via a first channel. In embodiments, the input is an electrical current of controlled voltage and frequency. In embodiments, the output is the inductance value of the inductance measurement circuit 749 with reference to the inductance measurement reference circuit 750.

The inductance measurement reference circuit 750 is configured to generate a reference inductance signal used to help measure the inductance in the inductance measurement circuit 749. In embodiments, the inductance measurement reference circuit 750 can be any suitable circuit 750 including the reference coil 751 and any other suitable components configured to generate a suitable reference inductance signal for comparative use with respect to the inductance value of the inductance measurement circuit 749. In embodiments, the reference coil 744 is connected to the power source 746 via a second channel which includes the inductance measurement reference circuit 750. In embodiments, the reference coil 751 is placed in offset relationship to measurement area 738 of the core 733. In embodiments, the reference coil 751 is placed in offset relationship to the measurement area 738 of the core 733 and is not wound around the core 733 itself. In embodiments, the reference coil 751 of the inductance measurement reference circuit 750 can be powered via a second channel of the power source 746.

In embodiments, the inductance measurement system 748 is configured to transmit an inductance signal from the inductance measurement circuit 749 via the first channel and a reference inductance signal from the inductance measurement reference circuit 750 via the second channel to a processor 769 programmed with a sensor debris detection program contained on a non-transitory computer readable medium 770. In embodiments, the inductance signal corresponds to the inductance value of the inductance measurement circuit 749, and the reference inductance signal corresponds to the inductance value of the inductance measurement reference circuit 750. The sensor debris detection program can be configured to provide any of the detection features described above in connection with other embodiments following principles of the present disclosure using both the inductance value of the inductance measurement circuit 749 and the inductance value of the inductance measurement reference circuit 750 based upon the inductance signal and the reference inductance signal. For example, in embodiments, the sensor debris detection program can be configured to provide a metallic debris detection feature described above using the difference between the inductance signal and the reference inductance signal. Any suitable processor for the purposes described herein can be used as will be appreciated by one skilled in the art. In embodiments, the metallic debris sensor assembly 727 of FIG. 16 can be similar in other respects to the metallic debris sensor assembly 327 of FIG. 9.

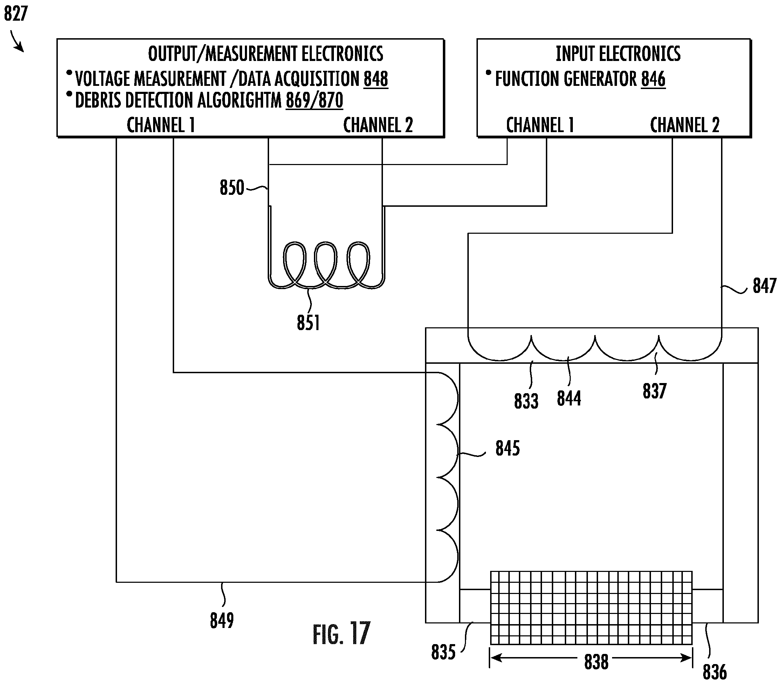

FIG. 17 is a schematic view of another embodiment of a metallic debris sensor assembly 827 constructed in accordance with principles of the present disclosure which includes a coil drive circuit 847 with a drive coil 844, an inductance measurement circuit 849 with a pickup coil 845, and an inductance measurement reference circuit 850 with a reference coil 851.

The pickup coil 845 is wound around the intermediate portion 837 of the core 833. In embodiments, the drive coil 844 is in substantial non-overlapping relationship with the pickup coil 845 along the intermediate portion 837 of the core 833. In embodiments, the drive coil 844 (the power coil or input coil) is connected to a power source 846 via a second channel including the coil drive circuit 847 (the power circuit or input circuit).

In embodiments, the inductance value is determined using the pickup coil 845 to enhance the sensitivity of the metallic debris sensor assembly 827 as compared to a metallic debris sensor assembly comprising a single coil in both the coil drive circuit and the inductance measurement circuit. In embodiments, the pickup coil 845 (a passive coil or output coil) is connected to an inductance measurement system 848 via a first channel including the inductance measurement circuit 849 which does not include the drive coil 844. In embodiments, the inductance value of the inductance measurement circuit 849 is variable based on an amount of current induced in the pickup coil 845 based upon the amount of magnetically responsive debris in the measurement area 838 between the first and second ends 835, 836 of the core 833.

The inductance measurement reference circuit 850 is configured to generate a reference inductance signal used to help measure the inductance in the inductance measurement circuit 849. In embodiments, the inductance measurement reference circuit 850 can be any suitable circuit 850 including the reference coil 851 and any other suitable components configured to generate a suitable reference inductance signal for comparative use with respect to the inductance value of the inductance measurement circuit 849. In embodiments, the reference coil 851 is connected to the power source 846 via a first channel which includes the inductance measurement reference circuit 850. In embodiments, the reference coil 851 is placed in offset relationship to measurement area 838 of the core 833. In embodiments, the reference coil 851 is placed in offset relationship to measurement area 838 of the core 833 and is not wound around the core 833 itself. In embodiments, the reference coil 851 of the inductance measurement reference circuit 850 can be powered via a different channel of the power source 746.

In embodiments, the inductance measurement system 848 is configured to transmit an inductance signal from the inductance measurement circuit 849 via the first channel and a reference inductance signal from the inductance measurement reference circuit 850 via a second channel to a processor 869 programmed with a sensor debris detection program contained on a non-transitory computer readable medium 870. The second channel transmitting the reference inductance signal to the processor 869 is separate from the first channel transmitting the inductance signal thereto.

In embodiments, the inductance signal corresponds to the inductance value of the inductance measurement circuit 849, and the reference inductance signal corresponds to the inductance value of the inductance measurement reference circuit 850. The sensor debris detection program can be configured to provide any of the detection features described above in connection with other embodiments following principles of the present disclosure using both the inductance value of the inductance measurement circuit 849 and the inductance value of the inductance measurement reference circuit 850 based upon the inductance signal and the reference inductance signal received via the first and second channels, respectively. For example, in embodiments, the sensor debris detection program can be configured to provide a metallic debris detection feature described above using the difference between the inductance signal and the reference inductance signal. Any suitable processor for the purposes described herein can be used as will be appreciated by one skilled in the art. In embodiments, the metallic debris sensor assembly 827 of FIG. 17 can be similar in other respects to the metallic debris sensor assembly 327 of FIG. 14 and/or to the metallic debris sensor assembly 727 of FIG. 16.

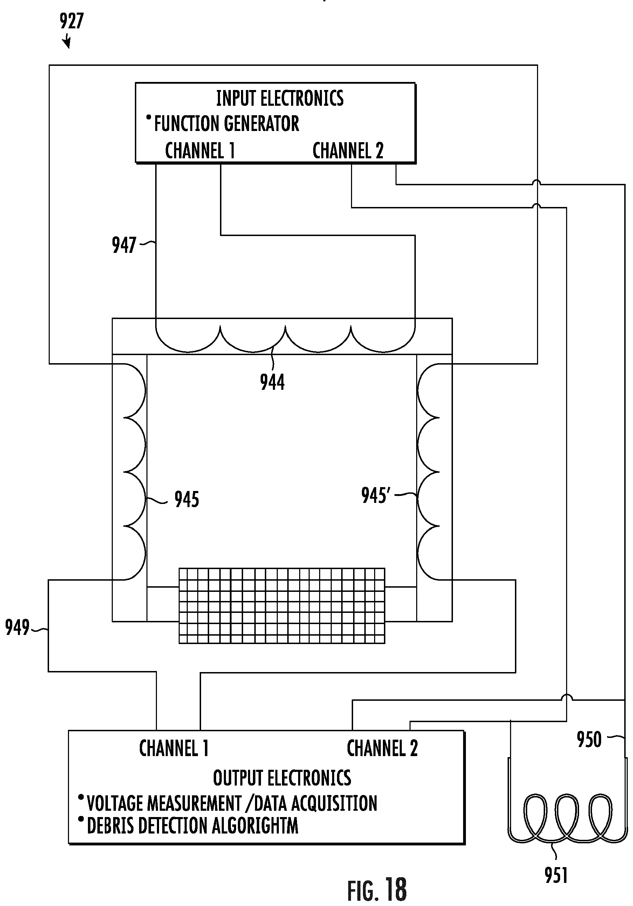

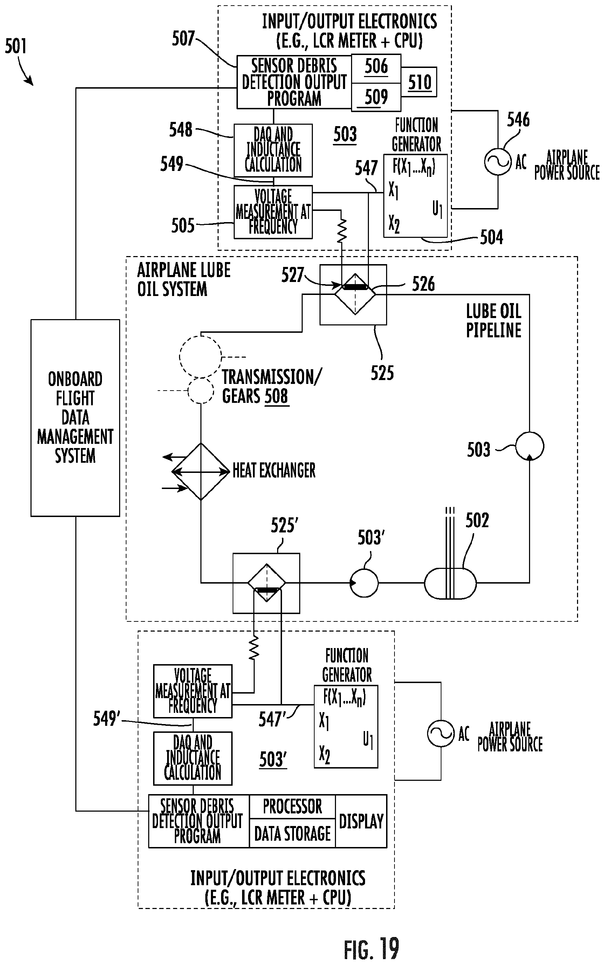

FIG. 19 is a schematic view of another embodiment of a metallic debris sensor assembly 927 constructed in accordance with principles of the present disclosure which includes a coil drive circuit 947 with a drive coil 944, an inductance measurement circuit 949 with a first pickup coil 945 and a second pickup coil 945', and an inductance measurement reference circuit 950 with a reference coil 951. In that respect, the metallic debris sensor assembly 927 of FIG. 18 is similar in other respects to the metallic debris sensor assembly 427 of FIG. 15 and/or to the metallic debris sensor assembly 827 of FIG. 18.

Embodiments of a wear detection system constructed according to principles of the present disclosure can include any embodiment of a filter constructed according to principles discussed herein. In embodiments, a wear detection system for monitoring wear of a part in a system of parts includes a filter for filtering particulates from a fluid stream that has a filter medium and a metallic debris sensor assembly constructed according to principles of the present disclosure as described above. In embodiments, the wear detection system comprises one or more parts (i.e., one part, two parts, three parts, four parts, or five parts) and one or more filters constructed according to principles of the present disclosure (i.e., one filter, two filters, three filters, four filters, or five filters).

Referring to FIG. 19, there is shown an embodiment of a wear detection system 501 constructed in accordance with principles of the present disclosure. The wear detection system 501 includes an upstream filter 525 and a downstream filter 525'. The upstream and downstream filters 525, 525' have substantially the same construction. Although only one filter will be described in detail, it should be understood that the other filter has a similar construction. In embodiments, the filter 525 includes a filter element 526 and a metallic debris sensor assembly 527 constructed according to principles of the present disclosure. In the illustrated embodiment, the wear detection system 501 is constructed as an on-board wear detection system installed in a mobile machine. An on-board wear detection system used in the manner described herein could be used for any suitable mobile machine, such as, e.g., aircraft, (airplanes and helicopters, e.g.), and machines configured to traverse over land. In other embodiments, the wear detection system can be implemented in other applications and have other arrangements.

Referring to FIG. 19, the wear detection system 501 can be used to monitor the wear of a part 508 in a system. In embodiments, such a part 508 could be a part within an airplane lube oil system such as a pump, transmission/gears, or a heat exchanger. However, the present invention is not limited to any particular system and can be used in any system in which a part 508 can be monitored for wear as described herein. In embodiments, the wear detection system 501 can be in operable communication with a larger management system of a mobile machine.

Referring to FIG. 19, the on-board wear detection system 501 includes a supply of fluid stored in a reservoir 502, first and second pumps 503, 503', at least one part 508 being monitored (e.g., the transmission/gears), upstream and downstream filters 525, 525' for filtering particulates from the flow of fluid and a respective coil drive circuit 547, 547' and an inductance measurement circuit 549, 549' associated with each filter 525, 525'. The fluid supply 502 can be any appropriate fluid that is known to a person skilled in the art to be located on a mobile machine. In embodiments, the pumps 503, 503' are each in fluid communication with the fluid supply which can be stored in the reservoir 502. In embodiments, the pumps 503, 503' are configured to draw the fluid supply into the pump 503 and to discharge a flow of fluid therefrom. In embodiments, the part 508 in the system is in fluid communication with the pump 503 to receive the flow of fluid. The other pump 503' can be provided to help draw fluid from the lube oil pipeline to return the fluid to the reservoir 502.

In embodiments, the filters 525, 525' can be any embodiment of a filter constructed according to the principles described herein. In embodiments, the on-board wear detection system 501 can include, for each filter 525, 525', a suitable coil drive circuit 547, 547' and inductance circuit 549, 549' according to principles described herein. In embodiments, the on-board wear detection system 501 further comprises a control unit 503, 503' associated with each filter 525, 525'.

As shown in FIG. 19, in embodiments, the coil drive circuit 547 includes a function generator 504. In embodiments, the function generator 504 is powered by an external AC power source 546. In embodiments, the function generator 504 is configured to provide variably frequency AC electrical current. The coil drive circuit 547' has substantially the same construction.

In embodiments, the inductance measurement circuit 549 includes any suitable inductance measurement system 548 configured to measure and calculate voltage, frequency, and inductance. As shown in FIG. 19, in embodiments, the inductance measurement circuit 549 can include an LCR meter 505. In embodiments, any suitable LCR meter 505 can be used, as will be appreciated by a person skilled in the art, for the purpose of determining inductance, capacitance, and resistance of the inductance measurement circuit according to principles described herein. The inductance measurement circuit 549' has substantially the same construction.

In embodiments, the control unit 503 is configured to selectively operate the metallic debris sensor assembly 527 associated with the filter 525 and to receive and analyze the inductance data received from the metallic debris sensor assembly 527. In the embodiment illustrated in FIG. 19, there are separate control units 503, 503' associated with the upstream and downstream filters 525, 525' which have substantially the same construction. It should be understood that, in embodiments, a single control unit can be associated with all of the filters of the wear detection system 501.

The control unit 503 includes a processor 506 and a non-transitory computer readable medium 507 which has stored thereon a sensor debris detection program. The processor 506 is arranged with the coil drive circuit 547 such that the processor 506 can control the operation of the coil drive circuit 547 via a coil drive module of the sensor debris detection program 507. The processor 506 is in electrical communication with the inductance measurement circuit 549 to receive the inductance signal, which is the inductance value of the inductance measurement circuit 549. In embodiments, the inductance value is measured by the inductance measurement system 548.

In embodiments, the processor 506 is programmed with the sensor debris detection program 507. In embodiments, the sensor debris detection program 507 has a wear characteristic determination module. In embodiments, the wear characteristic determination module is configured to determine a wear characteristic of a part 508 in the system of parts based upon the inductance signal. In embodiments, the wear characteristic is related to the amount of magnetically responsive debris in the measurement area of the metallic debris sensor assembly 527.

In embodiments, the wear detection system 501 further comprises a data storage device 509. The data storage device 509 is in communication with the processor 506. In embodiments, the data storage device 509 has a database of inductance values correlated with a respective time at which each inductance value was determined.

In embodiments, the sensor debris detection program 507 has a data interaction module. In embodiments, the data interaction module is configured to interrogate the database 509 to select a first inductance value determined at a first time and a second inductance value determined at a second time. In embodiments, the second time is prior to the first time.

In embodiments, the sensor debris detection program 507 includes a wear calculation module. In embodiments, the wear calculation module is configured to determine a debris generation rate. The debris generation rate can be determined via determining a difference in the amount of magnetically responsive debris detected in the measurement area of the metallic debris sensor assembly 527 at the first time relative to the amount of magnetically responsive debris in the measurement area detected at a second time. The amount of magnetically responsive debris is based on the inductance value of the inductance circuit 549 measured at the first and second times.

In embodiments, the sensor debris detection program 507 is configured to track output inductance over time such that the inductance change over time at a specific input can be compared. For example an inductance measurement is recorded with a clean filter media in the sensing area. Then metallic debris, such as iron particles, are introduced to the sensing area filter media and another inductance measurement is recorded. The change, or "delta," in those measurments can be correlated to an amount of magnetically responsive debris that has accumulated in the time between measurements.

In embodiments, the control unit 503 includes a display device 510 adapted to display a graphical user interface. Any suitable display device and graphical user interface can be used. A person skilled in the art will readily understand an appropriate display device and graphical user interface for the purpose of displaying results according to the principles described herein. In embodiments, the sensor debris detection program 507 includes an interface module, which is configured to display, through the graphical user interface, debris generation data in the display device 510.

In embodiments, the interface module of the sensor debris detection program 507 can generate at least one of a warning signal when the debris generation rate exceeds a failure threshold and a maintenance alert signal when the amount of debris generated exceeds a maintenance threshold. In embodiments, the warning signal and maintenance alert signal are generated through the graphical user interface. In embodiments, the warning signal and the maintenance alert signal are displayed on the display device. In embodiments, the warning signal comprises at least one of a visual indicator (e.g., a flashing light) or audible indicator (e.g., an audible beeping noise). In embodiments, the warning signal is conveyed to a remote device such as an engine data analytics hub, such as those operated for maintenance tracking, or to a remote operator location.

In embodiments, the sensor debris detection program 507 includes a part identification module, which can be configured to conduct a debris material frequency response analysis as described herein. The illustrated coil drive circuit 547 is configured to provide electrical current via an AC power source 546 with a variable frequency. The debris material frequency response analysis can be used to identify at least one part of the system of parts generating magnetically response debris.

In embodiments, the frequency of the AC power source 546 can be varied to determine whether the debris trapped by the filter has a measurable response with a frequency in a given range, such as less than 100 Hz or in a range between 100 Hz and 5000 Hz, for example. For example, aluminum chips (paramagnetic but electrically conductive) can have stronger measurable responses at frequencies less than 100 Hz while ferromagnetic debris such as iron chips or magnetite chips can have relatively low response magnitudes at such frequency range. Ferromagnetic debris can have greater measurable responses in a frequency range between 100 Hz and 5000 Hz while the aluminum chips can have lower responses in that frequency range. In embodiments, differences in measured inductance changes can be tracked when controlling for frequency, position, and mass of the test debris to help identify the material of the test debris. For example, at 100Hz, iron chips can produce greater change in inductance than magnetite.

In embodiments, a method of monitoring for wear in a system of parts comprises conducting a debris material frequency response analysis to identify at least one part of the system of parts generating magnetically responsive debris. In embodiments, a frequency range can be established (based on core material magnetic properties) such that the drive circuit input AC power frequency can be varied within the range in order to conduct a debris material frequency response analysis to identify the debris material. The induction response magnitude at a given frequency can correspond to the debris material characteristics, such that the debris material can be identified. In embodiments, the debris material characteristics are stored in the data storage device. In embodiments, the debris material is identified, leading to identification of the part in the system of parts experiencing wear. In embodiments, the sensor debris detection program has a data interaction module. In embodiments, the data interaction module is configured to interrogate the database to identify the at least one part of the system generating magnetically responsive debris.

In embodiments, the processor has a sensor debris detection program as described herein. In embodiments, the sensor debris detection program can determine a wear characteristic as described herein. In embodiments, the on-board wear detection system further comprises a data storage device as described herein. In embodiments, the sensor debris detection program has a data interaction module as described herein. In embodiments, the on-board wear detection system has a coil drive circuit which is configured to provide the electrical via an AC power source with a variable frequency as described herein. In embodiments, the on-board wear detection system can identify at least one part of the system generating magnetically responsive debris based on a debris material frequency response analysis as described herein.

Embodiments of a filter constructed according to principles of the present disclosure can be used to carry out a method of monitoring for wear of a part in a system of parts following principles of the present disclosure as described above. In embodiments, a method using a filter following principles of the present disclosure can be used with any embodiment of a filter constructed according to principles discussed herein.

In embodiments of a method of monitoring for wear of a part in a system of parts, at least one part in a system of parts is contacted with fluid. If the part is experiencing wear, the part can shed metallic particles, such that the part is worn down. The metallic particles can become entrained in the fluid after the fluid contacts the part. The fluid is passed in a direction of flow through a filter medium of a filter element as described herein. A metallic debris sensor assembly as described herein is operated to generate a magnetic field in a measurement area 38 including therein an upstream portion of the filter medium relative to the direction of flow according to the principles described herein. In embodiments, the metallic debris sensor assembly comprises a drive coil of electrically-conductive wire being wound around the intermediate portion of the core. An electrical current is passed through the drive coil and an inductance value of an inductance measurement circuit is determined. In embodiments, the inductance measurement circuit is arranged with the metallic debris sensor assembly such that the inductance value of the inductance measurement circuit is variable based upon an amount of magnetically responsive debris in the measurement. A wear characteristic of the part can then be determined based on upon the inductance value.

In embodiments, the coil drive circuit and the inductance measurement circuit both include the drive coil and the inductance value is determined using the drive coil. An example of this embodiment of a metallic debris sensor assembly is shown in FIG. 9. In embodiments, the metallic debris sensor assembly includes a pickup coil of electrically-conductive wire as described herein. In embodiments, the inductance measurement circuit includes the pickup coil, and the inductance value is determined using the pickup coil. In embodiments, the inductance value of the inductance measurement circuit is variable based upon an amount of current induced in the pickup coil based upon the amount of magnetically responsive debris in the measurement area 38. An example of this metallic debris sensor assembly is shown in FIG. 14. In embodiments, the metallic debris sensor assembly includes a first pickup coil and a second pickup coil as described herein. In embodiments, the inductance measurement circuit includes the first pickup coil and the second pickup coil, and the inductance value is determined using at least one of the first pickup coil and the second pickup coil. In embodiments, the inductance value is determined by the first pickup coil and the second pickup coil. In embodiments, the inductance value of the inductance measurement circuit is variable based upon a first amount of current induced in the first pickup coil based upon the amount of magnetically responsive debris in the measurement area and a second amount of current induced in the second pickup coil based upon the amount of magnetically responsive debris in the measurement area. An example of this metallic debris sensor assembly is shown in FIG. 15.

In embodiments, determining the wear characteristic of the part based upon the inductance value includes transmitting an inductance signal to a processor, the inductance signal corresponding to the inductance value of the inductance measurement circuit. Any suitable processor can be used according to the principles described herein of transmitting signals. In embodiments, determining the wear characteristic of the part based upon the inductance value also includes executing a sensor debris detection program stored upon a non-transitory computer-readable medium using the processor to generate the wear characteristic. In embodiments, the wear characteristic is related to the amount of magnetically responsive debris in the measurement area 38 based upon the inductance signal. Any suitable non-transitory computer-readable medium can be used according to the principles described herein.

In embodiments, the method of monitoring for wear of a part in a system of parts further comprises at least periodically determining the inductance value of the inductance measurement circuit. In embodiments, periodically determining the inductance value comprises determining the inductance value more than once over a period of time. In embodiments, a sensor debris detection program is then executed using the processor to store in a data storage device associated with the processor the inductance values that have been determined at least periodically correlated with the respective time at which each inductance value was determined. Any suitable data storage device can be used according to the principles described herein. A person skilled in the art would readily understand suitable data storage devices. The processer then can determine a difference in the amount of magnetically responsive debris detected in the measurement area 38 at a first time relative to the amount of magnetically responsive debris in the measurement area 38 detected at a second time, the second time being prior to the first time. A debris generation rate over time can then be determined. In embodiments, the debris generation rate can inform the part wear characteristic.

In embodiments, the sensor debris detection program can also use the processor to display, through a graphical user interface, debris generation data in a display device. Any suitable graphical user interface or display device can be used according to the principles described herein. In embodiments, the debris detection program can generate a warning signal when the debris generation rate exceeds a threshold. In embodiments, the warning signal is displayed on the display device using a graphical user interface. In embodiments, a maintenance alert can also be generated. In embodiments, the maintenance alert is displayed on the display device using a graphical user interface.

In embodiments, the method of monitoring for wear in a system of parts comprises conducting a debris material frequency response analysis to identify at least one part of the system of parts generating magnetically responsive debris. In embodiments, a frequency range can be established (based on core material magnetic properties) such that the drive circuit input AC power frequency can be varied within the range in order to conduct a debris material frequency response analysis to identify the debris material. The induction response magnitude at a given frequency will correspond to the debris material characteristics, such that the debris material can be identified. In embodiments, the debris material is identified, leading to identification of the part in the system of parts experiencing wear.

In embodiments, the debris material frequency response analysis includes: varying the frequency of the AC powering according to a predetermined protocol, such as a frequency range based on core material magnetic properties, transmitting a variable frequency inductance signal to a processor, the variable frequency inductance signal corresponding to the inductance value of the inductance measurement circuit during the predetermined protocol, executing a sensor debris detection program stored upon a non-transitory computer-readable medium using the processor to identify the at least one part of the system of parts generating magnetically responsive debris based upon the variable frequency inductance signal.

In embodiments, identifying the at least one part of the system of parts generating magnetically responsive debris based upon the variable frequency inductance signal includes corresponding an induction response magnitude at a given frequency to a part of the system by querying a data storage device containing a database of correlated material frequency induction responses for each part of the system of parts. In embodiments, the data storage device is prepopulated with material frequency induction responses for each material that makes up each part of the system of parts.

In embodiments, fluid passes through a system that contains parts. Over time, the parts wear, and metallic debris particles are released into the fluid. In embodiments, the fluid passes through the filter, and specifically the measurement area 38, optionally as illustrated in FIG. 9, and the metallic debris particles are collected in the measurement area 38.

In embodiments, the metallic debris sensor assembly comprises a coil of electrically-conductive wire as described herein. In embodiments, each end of the coiled wire has a sufficient length to connect to an electrical circuit connector for connecting the metallic debris sensor assembly to an external power source and an inductance measurement system. In embodiments, the external power source runs an alternating electrical current through the coil, and a magnetic field is induced in the measurement area 38. In embodiments, a single coil can be both the drive coil and the sensor coil. In other embodiments, a drive coil and at least one pickup coil for the inductance measurement circuit can be used.

In embodiments, the inductance measurement system can measure the inductance of the circuit and output the inductance to a processor programmed with a sensor debris detection program stored upon a non-transitory computer-readable medium and configured to monitor, record, and analyze the output electrical parameters (e.g., inductance, resistance, frequency response, etc.). These electrical response variables of the inductance measurement circuit change as magnetically susceptible debris accumulates within the measurement area 38 (system filter).

In embodiments, the sensor debris detection program can be configured to track the output inductance over time such that the inductance change over time at a specific input can be compared. In one embodiment, an inductance measurement is recorded with a clean filter media 29 in the measurement area. Then, metallic debris, for example, iron particles, are introduced to the measurement area and another inductance measurement is recorded. The change in the inductance measurements can be correlated to an amount of magnetically responsive debris that has accumulated in the time between measurements. In embodiments, inductance, resistance, and frequency response are measured by the inductance measurement system. In embodiments, the processor provides via a user interface device a data readout that tracks the metallic debris collection rate within a given system by tracking the induction change of the electrical circuit over time. The induction change can be continually compared back to a baseline that is established. In embodiments, if the generation rate begins to exceed a certain threshold, the processor could send a signal or message to indicate that a part is exiting the "normal wear phase" and entering a "severe wear phrase," which can indicate that maintenance may be required.

In embodiments, a frequency range can be established (based on core material magnetic properties) such that the input power frequency can be varied within the frequency range to conduct a debris material frequency response analysis to identify the debris material. The induction response magnitude at a given frequency can correspond to the debris material characteristics. In embodiments, identification of the debris material can be used to identify the part experiencing wear.

Embodiments of a filter 25 constructed according to principles of the present disclosure can be used to carry out a method of monitoring a fluid for metallic particles. In embodiments, a method using a filter 25 following principles of the present disclosure can be used with any embodiment of a filter 25 constructed according to principles discussed herein.

In embodiments, a fluid is passed in a direction of flow through a filter medium 29 of a filter element 26 as described herein. A metallic debris sensor assembly is operated according to the principles described herein, including generating a magnetic field in a measurement area 38 including therein an upstream portion of the filter medium 29 relative to the direction of flow. In embodiments, the metallic debris sensor assembly comprises a coil of electrically-conductive wire which is a drive coil, and the metallic debris sensor assembly operates through passing an electric current through a coil drive circuit including the drive coil. An inductance value of an inductance measurement circuit is determined as described according to the principles herein. The amount of magnetically responsive debris in the measurement area 38 can be determined based upon the inductance value.

In embodiments, the coil drive circuit and the inductance measurement circuit both include the drive coil and the inductance value is determined using the drive coil. An example of this embodiment of a metallic debris sensor assembly is shown in FIG. 9. In embodiments, the metallic debris sensor assembly includes a pickup coil of electrically-conductive wire as described herein. In embodiments, the inductance measurement circuit includes the pickup coil, and the inductance value is determined using the pickup coil. In embodiments, the inductance value of the inductance measurement circuit is variable based upon an amount of current induced in the pickup coil based upon the amount of magnetically responsive debris in the measurement area 38. An example of this metallic debris sensor assembly is shown in FIG. 14. In embodiments, the metallic debris sensor assembly includes a first pickup coil and a second pickup coil as described herein. In embodiments, the inductance measurement circuit includes the first pickup coil and the second pickup coil, and the inductance value is determined using at least one of the first pickup coil and the second pickup coil. In embodiments, the inductance value is determined by the first pickup coil and the second pickup coil. In embodiments, the inductance value of the inductance measurement circuit is variable based upon a first amount of current induced in the first pickup coil based upon the amount of magnetically responsive debris in the measurement area 38 and a second amount of current induced in the second pickup coil based upon the amount of magnetically responsive debris in the measurement area 38. An example of this metallic debris sensor assembly is shown in FIG. 15.

In embodiments, determining the amount of magnetically responsive debris in the measurement area 38 includes transmitting an inductance signal to a processor, the inductance signal corresponding to the inductance value of the inductance measurement circuit. Any suitable processor can be used according to the principles described herein of transmitting signals. In embodiments, determining the amount of magnetically responsive debris in the measurement area 38 based upon the inductance value also includes executing a sensor debris detection program stored upon a non-transitory computer-readable medium using the processor to generate a numerical value. In embodiments, the numerical value is related to the amount of magnetically responsive debris in the measurement area 38 based upon the inductance signal. Any suitable non-transitory computer-readable medium can be used according to the principles described herein.

In embodiments, the method of monitoring a fluid for metallic particles further comprises at least periodically determining the inductance value of the inductance measurement circuit. In embodiments, periodically determining the inductance value comprises determining the inductance value more than once over a period of time. In embodiments, a sensor debris detection program is then executed using the processor to store in a data storage device associated with the processor the inductance values that have been determined at least periodically correlated with the respective time at which each inductance value was determined. Any suitable data storage device can be used according to the principles described herein. A person skilled in the art would readily understand suitable data storage devices to be used. The processer then can determine a difference in the amount of magnetically responsive debris detected in the measurement area 38 at a first time relative to the amount of magnetically responsive debris in the measurement area 38 detected at a second time, the second time being prior to the first time. A debris generation rate over time can then be determined.

In embodiments, the method of monitoring a fluid for metallic particles comprises conducting a debris material frequency response analysis to identify at least one part of the system of parts generating magnetically responsive debris. In embodiments, a frequency range can be established (based on core material magnetic properties) such that the drive circuit input AC power frequency can be varied within the range in order to conduct a debris material frequency response analysis to identify the debris material. The induction response magnitude at a given frequency will correspond to the debris material characteristics, such that the debris material can be identified. In embodiments, the debris material is identified, leading to identification of the part in the system of parts experiencing wear.

In embodiments, the debris material frequency response analysis includes: varying the frequency of the AC powering according to a predetermined protocol, such as a frequency range based on core material magnetic properties, transmitting a variable frequency inductance signal to a processor, the variable frequency inductance signal corresponding to the inductance value of the inductance measurement circuit during the predetermined protocol, executing a sensor debris detection program stored upon a non-transitory computer-readable medium using the processor to identify the at least one part of the system of parts generating magnetically responsive debris based upon the variable frequency inductance signal.

In embodiments, identifying the magnetically responsive debris based upon the variable frequency inductance signal includes corresponding an induction response magnitude at a given frequency to a part of the system by querying a data storage device containing a database of correlated material frequency induction responses for each material of the parts being monitored that are magnetically responsive. In embodiments, the data storage device is prepopulated with material frequency induction responses for each material of the parts being monitored that are magnetically responsive.

In embodiments, the method includes determining a reference inductance value of an inductance reference circuit including a reference coil. The reference coil is placed in offset relationship to the measurement area of the core. The amount of magnetically responsive debris in the measurement area is determined based upon the inductance value, which includes comparing the difference between the inductance value and the reference inductance value.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non]-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A wear detection system for monitoring wear of a part in a system of parts, the wear detection system comprising:
a filter for filtering particulates from a fluid stream, the filter comprising:
a filter element, the filter element including a filter medium, and
a metallic debris sensor assembly, the metallic debris sensor assembly including a core and a drive coil of electrically-conductive wire, the core having a first end, a second end, and an intermediate portion interposed between the first and second ends, the first end disposed in spaced relationship with the second end such that a measurement area is disposed therebetween, the drive coil of electrically-conductive wire being wound around the intermediate portion of the core, the core being adapted to generate a magnetic field in the measurement area when an electrical current is passed through the drive coil,
wherein at least a portion of the filter medium is disposed within the measurement area;
a coil drive circuit operably arranged with the drive coil and configured to selectively provide the electrical current to the drive coil;
an inductance measurement circuit configured to determine an inductance value of the inductance measurement circuit, the inductance measurement circuit operably arranged with the metallic debris sensor assembly such that the inductance value of the inductance measurement circuit is variable based upon an amount of magnetically responsive debris in the measurement area.

2. The wear detection system according to claim 1, wherein the coil drive circuit and the inductance measurement circuit both include the drive coil.

3. The wear detection system according to claim 1, wherein the metallic debris sensor assembly includes a pickup coil of electrically-conductive wire, the pickup coil wound around the intermediate portion of the core, wherein the inductance measurement circuit includes the pickup coil, and wherein the inductance value of the inductance measurement circuit is variable based upon an amount of voltage induced in the pickup coil based upon the amount of magnetically responsive debris in the measurement area; and/or
wherein the metallic debris sensor assembly includes a first pickup coil of electrically-conductive wire and a second pickup coil of electrically-conductive wire, the first pickup coil and the second pickup coil both wound around the intermediate portion of the core, and wherein the inductance measurement circuit includes the first pickup coil and the second pickup coil, and wherein the inductance value of the inductance measurement circuit is variable based upon a first amount of current induced in the first pickup coil based upon the amount of magnetically responsive debris in the measurement area and a second amount of current induced in the second pickup coil based upon the amount of magnetically responsive debris in the measurement area.

4. The wear detection system according to any one of claim 1 to 3, wherein the inductance measurement circuit is configured to generate an inductance signal, the inductance signal corresponding to the inductance value of the inductance measurement circuit, the wear detection system further comprising:
a control unit, the control unit including a processor and a non-transitory computer readable medium bearing a sensor debris detection program, the processor being in electrical communication with the inductance measurement circuit to receive the inductance signal therefrom, the processor programmed with the sensor debris detection program, the sensor debris detection program having a wear characteristic determination module configured to determine a wear characteristic of the part based upon the inductance signal, the wear characteristic relating to the amount of magnetically responsive debris in the measurement area.

5. The wear detection system according to claim 4, further comprising:
a data storage device, the data storage device in operable communication with the processor, the data storage device including a database of inductance values correlated with a respective time at which each inductance value was determined;
wherein the sensor debris detection program has a data interaction module configured to store in the database determined inductance values correlated with a respective time at which each inductance value was determined and to interrogate the database to select a first inductance value determined at a first time and a second inductance value determined at a second time, the second time being prior to the first time, and wherein the sensor debris detection program includes a wear calculation module configured to determine a difference in the amount of magnetically responsive debris detected in the measurement area at the first time relative to the amount of magnetically responsive debris in the measurement area detected at the second time to determine a debris generation rate.

6. The wear detection system according to claim 4 or claim 5, wherein the control unit includes a display device adapted to display a graphical user interface, the sensor debris detection program includes an interface module, the interface module configured to display, through the graphical user interface, debris generation data in the display device; preferably
wherein the interface module of the sensor debris detection program is configured to generate, through the graphical user interface, at least one of a warning signal when the debris generation rate exceeds a failure threshold and a maintenance alert signal when the amount of debris generated exceeds a maintenance threshold.

7. The wear detection system according to any one of claim 1 to claim 6, wherein the coil drive circuit is configured to provide the electrical current via an AC power source with a variable frequency, and wherein the sensor debris detection program includes a part identification module, the part identification module being configured to conduct a debris material frequency response analysis via the AC power source and to identify at least one part of the system generating magnetically responsive debris based upon the debris material frequency response analysis; preferably
wherein the debris material frequency response analysis comprises a predetermined protocol for varying the frequency of the AC power, and wherein the part identification module being is configured to identify the at least one part of the system generating magnetically responsive debris based upon a variable frequency inductance signal received from the inductance measurement circuit during the predetermined protocol.

8. The wear detection system according to claim 7, further comprising:
a data storage device, the data storage device in operable communication with the processor, the data storage device including a database of correlated material frequency induction responses for each part of the system;
wherein the sensor debris detection program has a data interaction module configured to interrogate the database to identify the at least one part of the system generating magnetically responsive debris by comparing the variable frequency inductance signal received from the inductance measurement circuit to the database of correlated material frequency induction responses for each part of the system.

9. The wear detection system according to claim 7 or claim 8, wherein the coil drive circuit includes a function generator configured to variable frequency AC electrical current.

10. The wear detection system according to any one of claim 1 to claim 9, further comprising:
an inductance measurement reference circuit with a reference coil.

11. An on-board wear detection system for monitoring wear of a part in a system of parts located on a mobile machine, the on-board wear detection system comprising:
a supply of fluid;
a pump, the pump in fluid communication with the supply of fluid, the pump being configured to draw the supply of fluid into the pump and to discharge a flow of fluid therefrom;
the part, the part in fluid communication with the pump to recive the flow of fluid therefrom;
a filter for filtering particulates from the flow of fluid, the filter in fluid communication with the pump and with the part, the filter interposed between the part and the pump such that the flow of fluid passes through the filter, the filter comprising:
a filter element, the filter element including a filter medium, and
a metallic debris sensor assembly, the metallic debris sensor assembly including a core and a drive coil of electrically-conductive wire, the core having a first end, a second end, and an intermediate portion interposed between the first and second ends, the first end disposed in spaced relationship with the second end such that a measurement area is disposed therebetween, the drive coil of electrically-conductive wire being wound around the intermediate portion of the core, the core being adapted to generate a magnetic field in the measurement area when an electrical current is passed through the drive coil,
wherein at least a portion of the filter medium is disposed within the measurement area;
a coil drive circuit operably arranged with the drive coil and configured to selectively provide the electrical current to the drive coil;
an inductance measurement circuit configured to determine an inductance value of the inductance measurement circuit, the inductance measurement circuit operably arranged with the metallic debris sensor assembly such that the inductance value of the inductance measurement circuit is variable based upon an amount of magnetically responsive debris in the measurement area.

12. The on-board wear detection system according to claim 11, wherein the coil drive circuit and the inductance measurement circuit both include the drive coil; and/or
wherein the metallic debris sensor assembly includes a pickup coil of electrically-conductive wire, the pickup coil wound around the intermediate portion of the core, wherein the inductance measurement circuit includes the pickup coil, and wherein the inductance value of the inductance measurement circuit is variable based upon an amount of current induced in the pickup coil based upon the amount of magnetically responsive debris in the measurement area; and/or
wherein the metallic debris sensor assembly includes a first pickup coil of electrically-conductive wire and a second pickup coil of electrically-conductive wire, the first pickup coil and the second pickup coil both wound around the intermediate portion of the core, and wherein the inductance measurement circuit includes the first pickup coil and the second pickup coil, and wherein the inductance value of the inductance measurement circuit is variable based upon a first amount of current induced in the first pickup coil based upon the amount of magnetically responsive debris in the measurement area and a second amount of current induced in the second pickup coil based upon the amount of magnetically responsive debris in the measurement area.

13. The on-board wear detection system according to claim 11 or claim 12, wherein the inductance measurement circuit is configured to generate an inductance signal, the inductance signal corresponding to the inductance value of the inductance measurement circuit, the on-board wear detection system further comprising:
a control unit, the control unit including a processor and a non-transitory computer readable medium bearing a sensor debris detection program, the processor being in electrical communication with the inductance measurement circuit to receive the inductance signal therefrom, the processor programmed with the sensor debris detection program, the sensor debris detection program having a wear characteristic determination module configured to determine a wear characteristic of the part based upon the inductance signal, the wear characteristic relating to the amount of magnetically responsive debris in the measurement area.

14. The on-board wear detection system according to claim 13, further comprising:
a data storage device, the data storage device in operable communication with the processor, the data storage device including a database of inductance values correlated with a respective time at which each inductance value was determined;
wherein the sensor debris detection program has a data interaction module configured to store in the database determined inductance values correlated with a respective time at which each inductance value was determined and to interrogate the database to select a first inductance value determined at a first time and a second inductance value determined at a second time, the second time being prior to the first time, and wherein the sensor debris detection program includes a wear calculation module configured to determine a difference in the amount of magnetically responsive debris detected in the measurement area at the first time relative to the amount of magnetically responsive debris in the measurement area detected at the second time to determine a debris generation rate.

15. The on-board wear detection system according to any one of claim 11 to claim 14, wherein the coil drive circuit is configured to provide the electrical current via an AC power source with a variable frequency, and wherein the sensor debris detection program includes a part identification module, the part identification module being configured to conduct a debris material frequency response analysis via the AC power source and to identify at least one part of the system generating magnetically responsive debris based upon the debris material frequency response analysis.
